# EUROPÄISCHE PATENTANMELDUNG

(11) **EP 1 762 265 A1**
(43) Veröffentlichungstag der Anmeldung: **14.03.2007**
(21) Anmeldenummer: 06017210.3
(22) Anmeldetag: 18.08.2006
(51) Int. Cl.: A61M 15/00

(54) **Vorrichtung zum Inhalieren eines Pulvers**

(30) Priorität: 13.09.2005 DE 102005043449
(71) Anmelder: Braunform GmbH, 79353 Bahlingen (DE)
(72) Erfinder: Beller, Klaus-Dieter, Dr. med. Dipl.-Ing., 79341 Kenzingen (DE)
(74) Vertreter: Goy, Wolfgang

(57) **Zusammenfassung**

Eine Vorrichtung zum Inhalieren eines Pulvers, welches sich in einer perforierbaren Kapsel (8) befindet, weist Dorne (4) zum Perforieren der Kapsel auf. Diese Dorne sind als Pyramiden mit dreieckiger Grundfläche ausgebildet.

## Beschreibung

Die Erfindung betrifft eine Vorrichtung zum Inhalieren eines Pulvers nach dem Oberbegriff des Anspruchs 1.

Die erfindungsgemäße Vorrichtung ist allgemein zum Einnehmen eines Pulvers gedacht. Darunter ist zum einen zu verstehen, daß das Pulver beispielsweise ein pulverförmiges Arzneimittel sein kann, welches vom Patienten inhaliert werden soll. Es ist zum anderen darunter auch zu verstehen, daß das Pulver beispielsweise ein Nahrungsergänzungsmittel ist, das von der Mundschleimhaut aufgenommen und gegebenenfalls heruntergeschluckt wird.

Bei den derzeit bekannten Pulverinhalatoren wird die das Pulver enthaltende Kapsel mit Nadeln oder Dornen perforiert, so daß die Einatemluft an der perforierten Kapsel vorbeiströmt und das Pulver mitreißt. Nur ein geringer Teil der Atemluft durchströmt dabei die Kapsel direkt. Eine Alternative zu den vorbeschriebenen Nadeln sowie Dornen stellt die Perforierung mittels runden Hohldornen dar, durch welche hindurch die Einatemluft geführt wird.

Bei allen Runddornen (seien sie massiv oder hohl) kommt es mit zunehmendem Durchmesser zu wachsender Splitterbildung bei der Kapsel. In keinem Fall kommt es bei den gängigen pharmazeutischen Kapselmaterialien zu einem sauberen, glattrandigen Ausschneiden oder Ausstechen der Öffnung. Damit ist keine Dichtigkeit zwischen der Kapsel und dem Dorn herstellbar. Somit kommt es bei den bekannten Perforationstechniken zu Splitterbildungen und nur zu kleinen Öffnungen. Eine definierte und reproduzierbare Öffnung der Kapsel ist mit den gängigen Methoden nicht möglich.

Dadurch werden aber die Luftkanalwiderstände häufig unzulässig hoch. Des weiteren ragen Blisterteile in das Lumen hinein. Der Vorgang der Kapselperforierung erzeugt - wie ausgeführt - Splitter aus dem Kapselmaterial, welche mittels eines Siebes abgefangen werden müssen. Dieses Sieb erhöht jedoch den Luftkanalwiderstand zusätzlich.

Weiterhin bleiben Anteile des Pulvers an Hinterfangungen haften und kommen in Ruhezonen der Strömung zu liegen, was zu Lasten der Dosiergenauigkeit geht.

Schließlich lassen sich mit den bekannten Bauformen die Kapseln nicht mit einem Atemzug entleeren.

Davon ausgehend liegt der Erfindung die **Aufgabe** zugrunde, eine Vorrichtung zum Inhalieren eines Pulvers der eingangs angegebenen Art mit einer verbesserten Perforation der Kapsel zu schaffen.

Die technische **Lösung** ist gekennzeichnet durch die Merkmale im Kennzeichen des Anspruchs 1.

Damit ist eine Dornkonfiguration zur sicheren Öffnung von Kapseln in Kapselinhalatoren geschaffen. Die Grundidee des erfindungsgemäßen Pulverinhalators besteht darin, daß die Kapsel nicht mehr zufallsmäßig perforiert oder angestochen wird, sondern die Kapsel wird mit dem pyramidenförmigen Dorn an jedem Kapselende durchstochen. Damit wird eine glatte und dichte, durch die Bauform des Dornes vorgegebene Öffnung geschaffen. Der Dorn in Pyramidenform erzeugt dabei eine splitterfrei, definierte und abdichtende Perforierung der Kapsel. Der Dorn schneidet paßgenau die Kapsel auf und verhindert eine Splitterbildung. Die paßgenaue Perforierung und Abdichtung bewirkt, daß der Luftstrom aerodynamisch geführt wird und keine Anteile des Pulvers in der Vorrichtung, d. h. in dem Luftkanal haften bleiben. Dies erhöht und optimiert die Dosiergenauigkeit. Die Kapseln lassen sich mit einem Atemzug entleeren, was die Dosierung reproduzierbar macht und die Effektivität der Applikation verbessert. Da bei der Perforation keine Splitter entstehen, ist kein Schutzsieb zwischen dem Mundstück und der Kapsel notwendig, was den Atmungswiderstand zusätzlich verringern würde. Somit lassen sich insgesamt gesehen die Nachteile der bekannten Pulverinhalatoren (was die Perforation der Kapsel betrifft) lösen. Erfindungsgemäß wird dies dadurch erreicht, daß statt eines runden Dorns ein pyramidenförmiger Dorn spezieller Konfiguration verwendet wird. Durch diesen pyramidenförmigen Dorn schneidet dieser beim Einführen die Kapsel auf, so daß die entstehenden Dreicksflächen ohne Splitterbildung aufgebogen werden. Die Kanten der Pyramide sind dabei als scharf geschliffene Schneidkanten ausgebildet. Gleichzeitig werden die Öffnungen in der Kapsel offengehalten, und es wird mit hoher Dichtigkeit das Einströmen von Seitenluft verhindert.

Vorzugsweise handelt es sich gemäß der Weiterbildung in Anspruch 2 bei der Pyramide um eine regelmäßige Pyramide. Darunter ist zu verstehen, daß die Grundfläche der Pyramide ein regelmäßiges Vieleck ist und der Fußpunkt der Höhe in den Mittelpunkt der Grundfläche fällt.

In einer bevorzugten Weiterbildung ist gemäß Anspruch 3 die Grundfläche der Pyramide ein Dreieck. Dadurch werden die optimalsten Ergebnisse erzielt. Handelt es sich bei der Grundfläche der Pyramide dagegen um ein Viereck oder gar um ein Fünfeck, werden die Ergebnisse schlechter. Zwangsläufig wird bei einem Viereck oder gar um ein Fünfeck die Gesamtöffnungsfläche der Ansauglöcher kleiner, und es kommt wieder zu Splitterbildungen und damit geringe Dichtigkeit, da man sich immer mehr der runden Form nähert.

Eine weitere bevorzugte Weiterbildung schlägt gemäß Anspruch 4 vor, daß die Seitenflächen der Pyramide eine leichte Hohlkehlung nach innen aufweisen. Die Hohlkehlung in der Größenordnung der 1 bis 5-fachen Kapselwandstärke verhindert, daß die freigeschnittene Dreieckfläche die Ansauglöcher verlegt. Die Form der Hohlkehle erhöht zusätzlich die Schneidfähigkeit der Schnittkanten der Pyramide.

Eine weitere Weiterbildung schlägt gemäß Anspruch 5 vor, daß die Basis der Pyramide achsparallele Flächen aufweist. Somit hat die Basis des pyramidenförmigen Dorns senkrechte Aufstellflächen in der Höhe von maximal 5 Kapselstärken. Dadurch werden die geschnittenen Flächen zurückgehalten und verhindern ein Verlegen der Ansaugflächen.

Die Weiterbildung gemäß Anspruch 6 schlägt als Dorn einen sogenannten Hohldorn vor. Vorzugsweise weisen sämtliche Seitenflächen der Pyramide wenigstens eine Durchgangsöffnung für den Luftstrom auf.

Dabei sind die Öffnungen gemäß der Weiterbildung in Anspruch 7 vorzugsweise achsparallel zur Kapsel ausgerichtet. Dadurch ergibt sich ein optimaler axialer Luftstrom durch die Kapsel hindurch.

Ein Ausführungsbeispiel einer erfindungsgemäßen Vorrichtung zum Inhalieren eines Pulvers wird nachfolgend anhand der Zeichnungen beschrieben. In diesen zeigt:
- Fig. 1: die Inhalationsvorrichtung im geschlossenen Zustand in Gebrauchsstellung;
- Fig. 2: eine Einzeldarstellung des Dorns in vergrößertem Maßstab.

Die Inhalationsvorrichtung weist ein im weitesten Sinne rohrförmiges Gehäuse 1 bestehend aus zwei Gehäuseteilen auf. Dabei bildet das - in der Zeichnung linke - Gehäuseteil das Mund- oder Nasenstück und das - in der Zeichnung rechte - Gehäuseteil den Lufteinlaß. Zum Inhalieren weisen die beiden Gehäuseteile axiale sowie konische Durchgangskanäle 2 auf.

Die einander zugewandten Stirnseiten der beiden Gehäuseteile weisen jeweils eine Aussparung auf. Diese bilden bei zusammengefügten Gehäuseteilen eine Kammer 3.

Schließlich sind in den beiden Gehäuseteilen zwei Dorne 4 in Form von Hohldornen angeordnet. Diese Dorne 4 gehen von den Durchgangskanälen 2 aus und münden mit ihren Spitzen in der Kammer 3 des Gehäuses 1.

Die Dorne 4 sind als reguläre Pyramiden mit einer dreieckigen Grundfläche ausgebildet. Der Dorn 4 besitzt dabei eine Grundfläche mit senkrechten Aufstellflächen 5, welche somit parallel zur Mittellängsachse des Gehäuses 1 ausgerichtet sind.

Die drei Seitenflächen 6 weisen jeweils eine leichte Hohlkehlung auf. Außerdem besitzt jede der drei Seitenflächen 6 jeweils eine achsparallele Öffnung 7.

### Die Funktionsweise ist wie folgt:

Mittels der vorbeschriebenen Inhalationsvorrichtung soll ein Patient die Möglichkeit haben, das in einer Kapsel 8 befindliche pulverförmige Arzneimittel (oder eine andere Substanz) inhalieren zu können.

Zur Inbetriebnahme der Inhalationsvorrichtung werden die miteinander verbundenen Gehäuseteile des Gehäuses 1 voneinander getrennt. Die Kapsel 8 wird in die Kammer 3 gelegt.

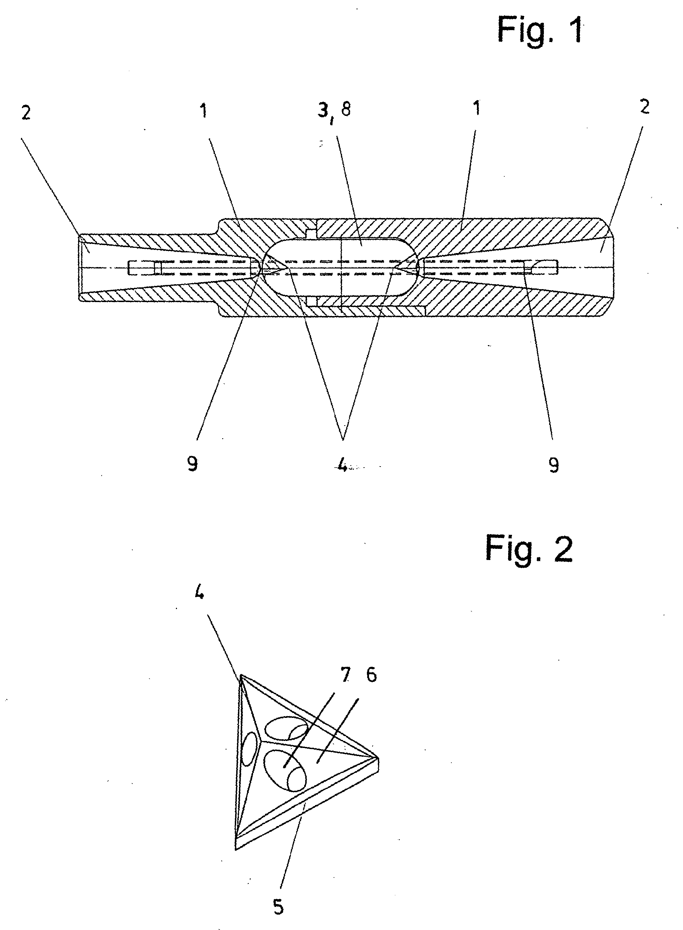

Zum Überführen des Pulverinhalators in die Gebrauchsstellung (Fig. 1) werden die beiden Gehäusehälften mittels eines Gestänges 9 zusammengeschoben. Dadurch werden die Kuppen der Kapsel 8 durch die pyramidenförmigen Dorne 4 perforiert. In der Endstellung befinden sich die Öffnungen 7 der Dorne 4 im Kapsellumen. Durch das exakte paßgenaue Perforieren der Kapsel 8 wird verhindert, daß im Seitschluß Luft angesaugt wird.

Die Längsachsen der Kapsel 8 sowie des Durchgangskanals 2 des Gehäuses 1 bilden eine gemeinsame Achse. Durch eine Inhalation durch den Benutzer wird das Pulver durch den Luftstrom aus dem Dorn 4 der Einlaßseite aerosolisiert, und durch den Dorn 4 der Mundstückseite wird das Aerosol abgesaugt und vom Patienten eingeatmet.

Das Inhalationsrohr kann dabei grundsätzlich entweder als Mundrohr oder als Nasenrohr konzipiert sein.

Nach der Inhalation wird das Gehäuse 1 auseinandergezogen und die komplett geleerte Kapsel 8 entfernt.

### Bezugszeichenliste

- 1: Gehäuse
- 2: Durchgangskanal
- 3: Kammer
- 4: Dorne
- 5: Aufstellflächen
- 6: Seitenflächen
- 7: Öffnungen
- 8: Kapsel
- 9: Gestänge

## Patentansprüche

1. Vorrichtung zum Inhalieren eines Pulvers,
wobei sich das Pulver in einer perforierbaren Kapsel (8) befindet,
die Vorrichtung
mit einem, einen axialen Durchgangskanal (2) aufweisenden Gehäuse (1) zur Aufnahme der Kapsel (8) in einer Kammer (3) sowie
mit zwei Dornen (4) zum Perforieren der Kapsel (8) an den gegenüberliegenden Enden der Kapsel (8) zur Schaffung eines durchgängigen Luftstroms,
**dadurch gekennzeichnet,**
**daß** die Dornen (4) pyramidenförmig ausgebildet sind.

2. Vorrichtung nach dem vorhergehenden Anspruch,
**dadurch gekennzeichnet,**
**daß** die Pyramide eine reguläre Pyramide ist.

3. Vorrichtung nach einem der vorhergehenden Ansprüche,
**dadurch gekennzeichnet,**
**daß** die Grundfläche Pyramide ein Dreieck ist.

4. Vorrichtung nach einem der vorhergehenden Ansprüche,
**dadurch gekennzeichnet,**
**daß** die Seitenflächen (6) der Pyramide eine Hohlkehlung nach innen aufweisen.

5. Vorrichtung nach einem der vorhergehenden Ansprüche,
**dadurch gekennzeichnet,**
**daß** die Basis der Pyramide achsparallele Aufstellflächen (5) aufweist.

6. Vorrichtung nach einem der vorhergehenden Ansprüche,
**dadurch gekennzeichnet,**
**daß** die Dorne (4) Hohldorne sind, wobei in wenigstens einer der Seitenflächen (6) wenigstens eine Öffnung (7) vorgesehen ist.

7. Vorrichtung nach Anspruch 6,
**dadurch gekennzeichnet,**
**daß** die Öffnung (7) achsparallel zur Kapsel (8) ausgerichet ist.
